# EUROPEAN PATENT SPECIFICATION

(11) **EP 1 344 773 B1**
(45) Date of publication and mention of the grant of the patent: **15.08.2007**
(21) Application number: 03075950.0
(22) Date of filing: 13.06.2001
(51) Int. Cl.: C07D 209/20, C07D 401/14, C07D 401/12, A61K 31/405

(54) **Growth hormone secretagogues**
Wachstumhormonsekretionsförderer
Secrétagogues de l'hormone de croissance

(30) Priority: 13.06.2000 US 211326 P; 26.09.2000 US 234928 P
(43) Date of publication of application: 17.09.2003
(62) Divisional of application: 01943504.9
(73) Proprietor: Zentaris GmbH, 60314 Frankfurt/Main (DE)
(72) Inventor: Fehrentz, Jean-Alain, 34400 St. Nazaire de Pezan (FR); Martinez, Jean, 34720 Le Jardin des Muses Caux (FR); Guerlavais, Vincent, Oakland, CA 94607 (US)
(74) Representative: Miles, John Stephen

(56) References cited:
- WO-A-95/14666
- WO-A-96/15148
- DATABASE CA [Online] CHEMICAL ABSTRACTS SERVICE, COLUMBUS, OHIO, US; DEGHENGHI, R. ET AL.: "Impervious peptides as growth hormone secretagogues" XP002178718 & PEPT. SCI.: PRESENT FUTURE, PROC. INT. PEPT. SYMP., 1ST, 1999, pages 411-412,
- PATCHETT A A ET AL: "DESIGN AND BIOLOGICAL ACTIVITIES OF L-163,191 (MK-0677): A POTENT, ORALLY ACTIVE GROWTH HORMONE SECRETAGOGUE" PROCEEDINGS OF THE NATIONAL ACADEMY OF SCIENCES OF USA, NATIONAL ACADEMY OF SCIENCE. WASHINGTON, US, vol. 92, no. 15, 1 July 1995 (1995-07-01), pages 7001-7005, XP000651085 ISSN: 0027-8424
- DATABASE CA [Online] CHEMICAL ABSTRACTS SERVICE, COLUMBUS, OHIO, US; MCDOWEEL, ROBERT S. ET AL.: "Growth hormone secretagogues" XP002178719 & PROCEEDINGS OF THE NATIONAL ACADEMY OF SCIENCES OF USA, vol. 92, no. 24, 1995, pages 11165-11169, WASHINGTON US

## Description

### FIELD OF THE INVENTION

The invention relates to compounds, which are useful for administration to a mammal thereby elevating the plasma level of growth hormone.

### BACKGROUND OF THE INVENTION

### (a) Description of Prior Art

Growth hormone (GH) or somatotropin, secreted by the pituitary gland constitute a family of hormones which biological activity is fundamental for the linear growth of a young organism but also for the maintenance of the integrity at its adult state. GH acts directly or indirectly on the peripheral organs by stimulating the synthesis of growth factors (insulin-like growth factor-I or IGF-I) or of their receptors (epidermal growth factor or EGF). The direct action of GH is of the type referred to as anti-insulinic, which favors the lipolysis at the level of adipose tissues. Through its action on IGF-I (somatomedin C) synthesis and secretion, GH stimulates the growth of the cartilage and the bones (structural growth), the protein synthesis and the cellular proliferation in multiple peripheral organs, including muscles and the skin. Through its biological activity, GH participates within adults at the maintenance of a protein anabolism state, and plays a primary role in the tissue regeneration phenomenon after a trauma.

The decrease of GH secretion with the age, demonstrated in humans and animals, favors a metabolic shift towards catabolism which initiates or participates to the ageing of an organism. The loss in muscle mass, the accumulation of adipose tissues, the bone demineralization, the loss of tissue regeneration capacity after an injury, which are observed in elderly, correlate with the decrease in the secretion of GH.

GH is thus a physiological anabolic agent absolutely necessary for the linear growth of children and which controls the protein metabolism in adults.

Growth hormone (GH) secretion is regulated by two hypothalamic peptides: OH-releasing hormone (GHRH), which exerts stimulatory effect on GH release and somatostatin which exhibits an inhibitory influence. In the last few years, several investigators have demonstrated that GH secretion can also be stimulated by synthetic oligopeptides termed GH-releasing peptides (GHRP) such as hexarelin and various hexarelin analogs (Ghigo et al., European Journal of Endocrinology, 136,445-460, 1997). These compounds act through a mechanism which is distinct from that of GHRH (C.Y. Bowers, in "Xenobiotic Growth Hormone Secretagogues", Eds. B.Bercu and R.F. Walker, Pg. 9-28, Springer-Verlag, New York 1996) and by interaction with specific receptors localized in the hypothalamus and pituitary gland ((a) G. Muccioli et al., Journal of Endocrinology, 157,99-106,1998 (b) G. Muccioli, "Tissue Distribution of GHRP. Receptors in Humans", Abstracts IV European Congress of Endocrinology, Sevilla, Spain, 1998). Recently it was demonstrated that GHRP receptors are present not only in the hypothalamo-pituitary system but even in various human tissues not generally associated with GH release (G. Mucci oli et al, see above (a)).

GHRPs and their antagonists are described, for example, in the following publications: C.Y. Bowers, supra, R. Deghenghi, "Growth Hormone Releasing Peptides", ibidern, 1996, pg. 85-102; R. Deghenghi et al., "Small Peptides as Potent Releasers of Growth Hormone", J. Ped. End. Metab., 8, pg. 311 -313, 1996; R. Deghenghi, "The Development of Impervious Peptides as Growth Hormone Secretagogues", Acta Paediatr. Suppl., 423, pg. 85-87,1997; K. Veeraraganavan et al., "Growth Hormone Releasing Peptides (GHRP) Binding to Porcine Anterior Pituitary and Hypothalamic Membranes", Life Sci., 50, Pg. 1149-1155, 1992; A.A Patchett et al., "Design and biological activities of L-163, 191 (MK-0677) : A patent, orally active growth hormone secretagogue", Proc. Nat. Acad. Sci. USA, 92, Pg. 7001-7005, 1995 ; R. Deghenghi et al., "Impervious peptides as growth hormone secretagogues", Pept. Sci : Present Future Proc. Int. Pept. Symp. 1st (1999), Editor : Y. Shimonishi ; M. Chen et.al, "Indolyl group containing compounds and the use thereof to promote the release of growth hormone(s)", WO 95/14666 and T.C. Somers et. al., "Low. Molecular Weight Peptidomimetic Growth Hormone Secretagogues, WO 96/15148 (May 23, 1996).

The human GH has been produced by genetic engineering for about ten years. Until recently most of the uses of GH Were concerned with growth delay in children amino now the uses of GH in adults are studied. The pharmacological uses of GH, GHRPs and growth hormone secretagogues and may be classified in the following three major categories.

### (b) Children growth

Treatments with recombinant human growth hormone have been shown to stimulate growth in children with pituitary dwarfism, renal insufficiencies, Turner's syndrome and short stature. Recombinant human GH is presently commercialized in Europe and in the United States for children's growth retardation caused by a GH deficiency and for children's renal insufficiencies. The other uses are under clinical trial investigation.

### (c) Long term treatment for adults and elderly patients

A decrease in GH secretion causes changes in body composition during aging. Preliminary studies of one-year treatment with recombinant human GH reported an increase in the muscle mass and in the thickness of skin, a decrease in fat mass with a slight increase in bone density in a population of aged patients. With respect to osteoporosis, recent studies suggest that recombinant human GH does not increase bone mineralization but it is suggested that it may prevent bone deinineralization in post-menopausal women. Further studies are currently underway to demonstrate this theory.

### (d) Short term treatment in adults and elderly patients

In preclinical and clinical studies, growth hormone has been shown to stimulate protein anabolism and healing in cases of burn, AIDS and cancer, in wound and bone healing.

GH, GHRPs and growth hormone secretagogues are also intended for veterinary pharmacological uses. GH, GHRPs and growth hormone secretagogues stimulate growth in pigs during its fattening period by favoring the deposition of muscle tissues instead of adipose tissues and increase milk production in cows, and this without any undesired side effects which would endanger the health of the animals and without any residue in the meat or milk being produced. The bovine somatotropin (BST) is presently commercialized in the United States.

Most of the clinical studies presently undertaken were conducted with recombinant GH. The GHRPs and growth hormone secretagogues are considered as a second generation product destined to replace in the near future the uses of GH in most instances. Accordingly, the use of GHRPs and growth hormone secretagogues present a number of advantages over the use of GH per se.

Therefore, there is a need for compounds which, when administered to a mammal, act as growth hormone secretagogues.

### SUMMARY OF THE INVENTION

The present invention relates to new compounds which act as growth hormone secretagogues and their use in a medicament for elevating the plasma level of growth hormone in a mammal by administering thereto one or more of the compounds according to the invention. The invention also relates to medicaments for the treatment of growth hormone secretion deficiency, for promoting wound healing, recovery from surgery or recovery from debilitating illnesses, by administering to a mammal one of these compounds in a therapeutically effective amount.

### DETAILED DESCRIPTION OF THE PREFERRED EMBODIMENTS

In this description, the following abbreviations are used: D is the dextro enantiomer, GH is growth hormone, Boc is tert-butyloxycarbonyl, Z is benzyloxycarbonyl, N-Me is N-methyl, Pip is 4-amino-piperidine-4-carboxylate, Inip is isonipecotyl, i.e. piperidine-4-carboxylate, Aib is α-amino isobutyryl, Nal is β-naphthylalanine, Mrp is 2-Methyl-Trp, and Ala, Lys, Phe, Trp, His, Thr, Cys, Tyr, Leu, Gly, Ser, Pro, Glu, Arg, Val and Gln are the amino acids alanine, lysine, phenylalanine, tryptophan, histidine, threonine, cysteine, tyrosine, leucine, glycine, serine, proline, glutamic acid, arginine, valine and ' glutamine, respectively. Furthermore gTrp is a group of the formula 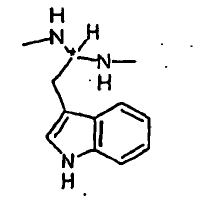 and gMrp a group of the formula 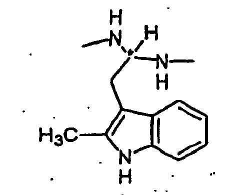 wherein * means a carbon atom which, when a chiral carbon atom, has a R or S configuration. The compounds of the invention are of the general formula I: 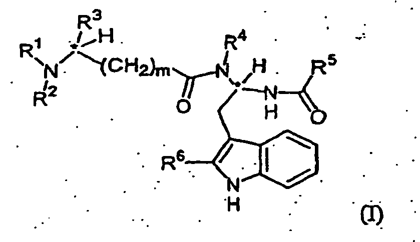 wherein * means a carbon atom which, when a chiral carbon atom, has a R or S configuration, R¹ is an hydrogen atom and R³ is a group of formula II 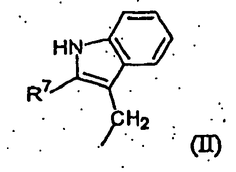 R² is a group according to formula IV below: 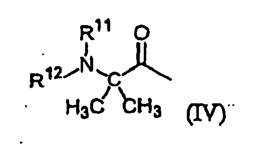 R⁴ is a hydrogen atom or a linear or branched C₁-C₄-alkyl group, R⁵ is a hydrogen atom, a linear or branched C₁-C₄alkyl group, a (CH₂)ₙ-aryl group, wherein aryl is phenyl or napthyl, a (CH₂)ₙ-heterocycle group, wherein the heterocyclic group is isonipecotyl, 4-piperidinyl or 3-pyrrolyl, a (CH₂)ₙ-cycloalkyl group, wherein the cycloalkyl group is cyclohexyl, or an amino group, R⁶ and R⁷ are independently from each other a hydrogen atom or a linear or branched C₁-C₄-alkyl group, R¹¹ and R¹² are independently from each other a hydrogen atom or a linear or branched C₁-C₄-alkyl group, m is 0 and n is 1 or 2.

A preferred embodiment of the invention are compounds, wherein linear or branched C₁-C₄ alkyl is methyl, linear or branched C₁-C₆ alkyl is methyl, ethyl or i-butyl, aryl is phenyl or naphthyl, cycloalkyl is cyclohexyl and the heterocyclic group is a 4-piperidinyl or 3-pyrrolyl group.

In accordance with the present invention, it has been found that the compounds of the invention are useful for the manufacture of a medicament for elevating the plasma level of growth hormone in a mammal. Furthermore the compounds of the present invention are useful for the manufacture of a medicament for the treatment of growth hormone secretion deficiency, growth retardation in child and metabolic disorders associated with growth hormone secretion deficiency, in particular in aged subjects.

Pharmaceutically acceptable salts of these compounds can be also used, if desired. Such salts include organic or inorganic addition salts, such as hydrochloride, hydrobromide, phosphate, sulfate, acetate, succinate, ascorbate, tartrate, gluconate, benzoate, malate, fumarate, stearate or pamoate salts.

Pharmaceutical compositions of the invention are useful for elevating the plasma level of growth hormone in a mammal, including a human, as well for the treatment of growth hormone secretion deficiency, growth retardation in child and metabolic disorders associated with growth hormone secretion deficiency, in particular in aged subjects. Such pharmaceutical compositions can comprise a compound according to the present invention or a pharmaceutically acceptable salt thereof, or combinations of compounds according to the present invention or pharmaceutically acceptable salts thereof, optionally in admixture with a carrier, excipient, vehicle, diluent, matrix; or delayed release coating. Examples of such carriers, excipients, vehicles, and diluents, can be found in Remington's Pharmaceutical Sciences, 18th Edition, A.R. Gennaro, Ed., Mack Publishing Company, Easton, P.A, 1990.

The pharmaceutical compositions of the invention can comprise an additional growth hormone secretagogue. Examples for suitable additional growth hormone secretagogues are Ghrelin (cf. M. Kojima et al., Nature, 402 (1999), 656-660), GHRP-1, GHRP-2 and GHRP-6.

| | |
|---|---|
| Ghrelin: | Gly-Ser-Ser(O-n-octanoyl)-Phe-Leu-Ser Pro-Glu-His-Gln-Arg-Val-Gln-Gln-Arg-Lys-Glu-Ser-Lys-Lys-Pro-Pro-Ala-Lys-Leu-Gln-Pro-Arg |
| GHRP-1: | Ala-His-D-β-Nal-Ala-Trp-D-Phe-Lys-NH₂ |
| GHRP-2: | D-Ala-D-β-Nal-Ala-Trp-D-Phe-Lys-NH₂ |
| GHRP-6: | His-D-Trp-Ala-Trp-D-Phe-Lys-NH₂ |

Any of the compounds according to the present invention can be formulated by the skilled in the art to provide medicaments which are suitable for parenteral, buccal, rectal, vaginal, transdermal, pulmonary or oral routes of administration.

The type of formulation of the medicament containing the compound can be selected according to the desired rate of delivery. For example, if the compounds are to be rapidly delivered, the nasal or intravenous route is preferred.

The medicaments can be administered to mammals, including humans, at a therapeutically effective dose which can be easily determined by one of skill in the art and which can vary according to the species, age, sex and weight of the treated patient or subject as well the route of administration. The exact level can be easily determined empirically.

### EXAMPLES

The following examples illustrate the efficacy of the most preferred compounds used in the treatment of this invention.

### Example 1: H-Aib-D-Trp-D-gTrp-CHO

Total synthesis (percentages represent yields obtained in the synthesis as described below): 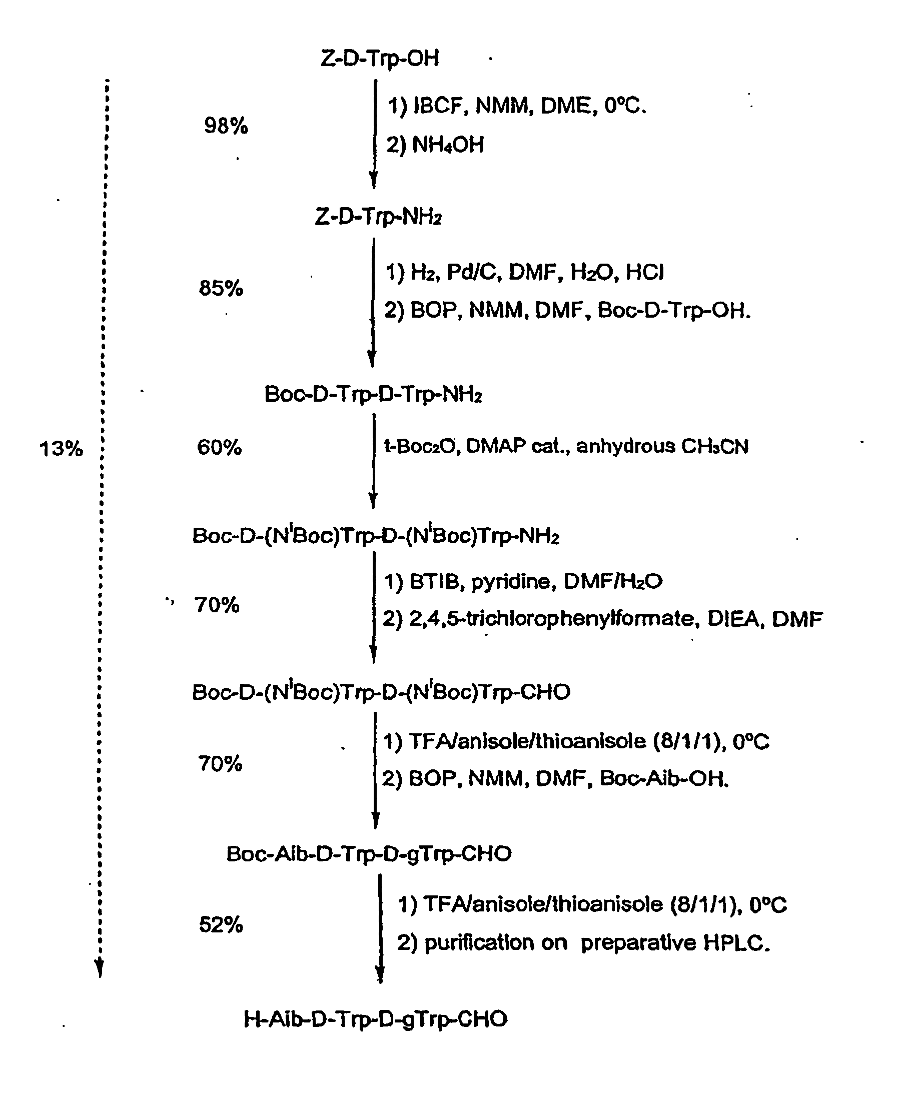 Z-D-Trp-NH₂

Z-D-Trp-OH (8.9g; 26mmol; 1eq.) was dissolved in DME (25ml) and placed in an ice water bath to 0°C. NMM (3.5ml; 1.2eq.), IBCF (4.1ml; 1.2eq.) and ammonia solution 28% (8.9ml; 5eq.) were added successively. The mixture was diluted with water (100ml), and the product Z-D-Trp-NH₂ precipitated. It was filtered and dried *in vacuo* to afford 8.58g of a white solid.
Yield = 98%.
C₁₉H₁₉N₃O₃, 337 g.mol⁻¹.
Rf = 0.46 {Chloroform/Methanol/Acetic Acid (180/10/5)}.
¹HNMR (250 MHZ, DMSO-d⁶) : δ 2.9 (dd, 1H, H_{β}, J_{ββ'} = 14.5Hz; J_{βα} = 9.8Hz); 3.1 (dd, 1H, H_{β'}, J_{β'β} = 14.5Hz; J_{β'α} = 4.3Hz); 4.2 (sextuplet, 1H, H_{α}); 4.95 (s, 2H; CH₂ (Z)); 6.9-7.4 (m, 11H); 7.5 (s, 1H, H²) ; 7.65 (d, 1H, J = 7.7Hz); 10.8 (s, 1H, N¹H).
Mass Spectrometry (Electrospray), m/z 338 [M+H]⁺, 360 [M+Na]⁺, 675 [2M+H]⁺, 697 [2M+Na]⁺.

### Boc-D-Trp-D-Trp-NH₂

Z-D-Trp-NH₂ (3g; 8.9mmol; 1eq.) was dissolved in DMF (100ml). HCl 36% (845 µl; 1.1 eq.), water (2ml) and palladium on activated charcoal (95mg, 0.1eq.) were added to the stirred mixture. The solution was bubbled under hydrogen for 24 hr. When the reaction went to completion, the palladium was filtered on celite. The solvent was removed *in vacuo* to afford HCl, H-D-Trp-NH₂ as a colorless oil.

In 10ml of DMF, HCl, H-D-Trp-NH₂ (8.9mmol; 1eq.), Boc-D-Trp-OH (2.98g; 9.8mmol; 1.1eq.), NMM (2.26ml; 2.1eq.) and BOP (4.33g; 1.1eq.) were added successively. After 1 hr, the mixture was diluted with ethyl acetate (100ml) and washed with saturated aqueous sodium hydrogen carbonate (200ml), aqueous potassium hydrogen sulfate (200ml, 1M), and saturated aqueous sodium chloride (100ml). The organic layer was dried over sodium sulfate, filtered and the solvent removed *in vacuo* to afford 4.35g of Boc-D-Trp-D-Trp-NH₂ as a white solid.
Yield = 85%.
C₂₇H₃₁N₅O₄, 489 g.mol⁻¹.
Rf= 0.48 {Chloroform/Methanol/Acetic Acid (85/10/5)}.
¹HNMR (200 MHZ, DMSO-d⁶): δ 1.28 (s, 9H, Boc); 2.75-3.36 (m, 4H, 2 (CH₂)_{β}; 4.14 (m, 1H, CH_{α}); 4.52 (m, 1H, CH_{α'}); 6.83-7.84 (m, 14H, 2 indoles (10H), NH₂, NH (urethane) and NH (amide)); 10.82 (d, 1H, J = 2Hz, N¹H); 10.85 (d,1H, J = 2Hz, N¹H).
Mass Spectrometry (Electrospray), m/z 490 [M+H]⁺, 512 [M+Na]⁺, 979 [2M+H]⁺.

### Boc-D-(NiBoc)Trp-D-(NiBoc)Trp-NH₂

Boc-D-Trp-D-Trp-NH₂ (3g; 6.13mmol; 1eq.) was dissolved in acetonitrile (25ml). To this solution, di-tert-butyl-dicarbonate (3.4g; 2.5eq.) and 4-dimethylaminopyridine (150mg; 0.2eq.) were successively added. After 1 hr, the mixture was diluted with ethyl acetate (100ml) and washed with saturated aqueous sodium hydrogen carbonate (200ml), aqueous potassium hydrogen sulfate (200ml, 1M), and saturated aqueous sodium chloride (200ml). The organic layer was dried over sodium sulfate, filtered and the solvent removed *in vacuo.* The residue was purified by flash chromatography on silica gel eluting with ethyl acetate/hexane {5/5} to afford 2.53g of Boc-D-(NⁱBoc)Trp-D-(NⁱBoc)Trp-NH₂ as a white solid.
Yield = 60%.
C₃₇H₄₇N₅O₈, 689 g.mol⁻¹.
Rf = 0.23 {ethyl acetate/hexane (5/5)}.
¹H NMR (200 MHZ, DMSO-d⁶) : δ1.25 (s, 9H, Boc); 1.58 (s, 9H, Boc); 1.61 (s, 9H, Boc); 2.75-3.4 (m, 4H, 2 (CH₂)_{β}); 4.2 (m, 1H, CH_{α'}); 4.6 (m, 1H, CH_{α}); 7.06-8 (m, 14H, 2 indoles (1 OH), NH (urethane), NH and NH₂ (amides)).
Mass Spectrometry (Electrospray), m/z 690 [M+H]⁺, 712 [M+Na]⁺; 1379 [2M+H]⁺, 1401 [2M+Na]⁺.

### Boc-D-(NⁱBoc)Trp-D-g(NⁱBoc)Trp-H

Boc-D-(NⁱBoc)Trp-D-(NⁱBoc)Trp-NH2 (3g; 43mmol; 1eq.) was dissolved in the mixture DMF / water (18ml / 7ml). Then, pyridine (772µl; 2.2eq.) and Bis(Trifluoroacetoxy)IodoBenzene (2.1g; 1.1eq.) were added. After 1 hr, the mixture was diluted with ethyl acetate (100ml) and washed with saturated aqueous sodium hydrogen carbonate (200ml), aqueous potassium hydrogen sulfate (200ml, 1M), and aqueous saturated sodium chloride (200ml). The organic layer was dried over sodium sulfate, filtered and the solvent removed *in vacuo.* Boc-D-(NⁱBoc)Trp-D-g(NⁱBoc)Trp-H was used immediately for the next reaction of formylation.
Rf = 0.14 {ethyl acetate/hexane (7/3)}.
C₃₆H₄₇N₅O₇, 661 g.mol⁻¹.
¹H NMR (200 MHZ, DMSO-d⁶) : δ 1.29 (s, 9H, Boc); 1.61 (s, 18H, 2 Boc); 2.13 (s, 2H, NH₂ (amine)); 3.1-2.8 (m, 4H, 2 (CH₂)_{β}) ; 4.2 (m,1H, CH_{α}); 4.85 (m,1H, CH_{α}) ; 6.9-8 (m, 12H, 2 indoles (10H), NH (urethane), NH (amide)).
Mass Spectrometry (Electrospray), m/z 662 [M+H]⁺, 684 [M+Na]⁺.

### Boc-D-(NⁱBoc)Trp-D-g(NⁱBoc)Trp-CHO

Boc-D-(NⁱBoc)Trp-D-g(NⁱBoc)Trp-H (4.3mmol; 1eq.) was dissolved in DMF (20ml). Then, N,N-diisopropylethylamine (815µl; 1.1eq.) and 2,4,5-trieblorophenylformate (1.08g; 1.1eq.) were added. After 30 minutes, the mixture was diluted with ethyl acetate (100ml) and washed with saturated aqueous sodium hydrogen carbonate (200ml), aqueous potassium hydrogen sulfate (200ml, 1M), and saturated aqueous sodium chloride (200ml). The organic layer was dried over sodium sulfate, filtered and the solvent removed *in vacuo.* The residue was purified by flash chromatography on silica gel eluting with ethyl acetate/hexane {5/5} to afford 2.07g of Boc-D-(NⁱBoc)Trp-D-g(NⁱBoc)Trp-CHO as a white solid.
Yield = 70%.
C₃₇H₄₇N₅O₈, 689 g.mol⁻¹.
Rf= 0.27 {ethyl acetate/hexane (5/5)}.
¹H NMR (200 MHZ, DMSO-d⁶): δ 1.28 (s, 9H, Boc); 1.6 (s, 9K Boc); 1.61 (s, 9H, Boc); 2.75-3.1 (m, 4H, 2 (CH₂)_{β}); 4.25 (m, 1H, (CH)α_{A&B}); 5.39 (m, 0.4H, (CH)α'_{B}); 5.72 (m, 0.6H, (CH)α'_{A}); 6.95-8.55 (m, 14H, 2 indoles (10H), NH (urethane), 2 NH (amides), CHO (formyl)).
Mass Spectrometry (Electrospray), m/z 690 [M+H]⁺, 712 [M+Na]⁺, 1379 [2M+H]⁺.

### Boc-Aib-D-Trp-D-gTrp-CHO

Boc-D-(NⁱBoc)Trp-D-g(NⁱBoc)Trp-CHO (1.98g; 2.9mmol; 1eq.) was dissolved in a mixture of trifluoroacetic acid (16ml), anisole (2ml) and thioanisole (2ml) for 30 minutes at 0°C. The solvents were removed *in vacuo,* the residue was stirred with ether and the precipitated TFA, H-D-Trp-D-gTrp-CHO was filtered.

TFA, H-D-Trp-D-gTrp-CHO (2.9mmol; 1eq.), Boc-Aib-OH (700mg; 1eq.), NMM (2.4ml; 4.2eq.) and BOP (1.53g; 1.2eq.) were successively added in 10ml of DMF. After 1 hr, the mixture was diluted with ethyl acetate (100ml) and washed with saturated aqueous sodium hydrogen carbonate (200ml), aqueous potassium hydrogen sulfate (200ml, 1M), and saturated aqueous sodium chloride (200ml). The organic layer was dried over sodium sulfate, filtered and the solvent removed *in vacuo.* The residue was purified by flash chromatography on silica gel eluting with ethyl acetate to afford 1.16g of Boc-Aib-D-Trp-D-gTrp-CHO as a white solid.
Yield = 70%.
C₃₁H₃₈N₆O₅, 574 g.mol⁻¹.
Rf= 0.26 {Chloroform/Methanol/ Acetic Acid (180/10/5)}.
¹H NMR (200 MHZ, DMSO-d⁶): δ 1.21 (s, 6H, 2 CH₃(Aib)); 1.31 (s, 9H, Boc); 2.98-3.12 (m, 4H, 2 (CH₂)_{β}); 4.47 (m, 1H, (CH)α_{A&B}); 5.2 (m, 0.4H, (CH)α'_{B}); 5.7 (m, 0.6H, (CH)α'_{A}); 6.95-8.37 (m, 15H, 2 indoles (10H), 3 NH (amides), 1 NH (urethane), CHO (formyl)); 10.89 (m, 2H, 2 N'H (indoles)).
Mass Spectrometry (Electrospray), m/z 575 [M+H]⁺, 597 [M+Na]⁺, 1149 [2M+H]⁺, 1171 [2M+Na]⁺.

### H-Aib-D-Trp-D-gTrp-CHO

Boc-Aib-D-Trp-D-gTrp-CHO (1g; 1.7mmol) was dissolved in a mixture of trifluoroacetic acid (8ml), anisole (1ml) and thioanisole (1ml) for 30 minutes at 0°C. The solvents were removed *in vacuo,* the residue was stirred with ether and the precipitated TFA, H-Aib-D-Trp-D-gTrp-CHO was filtered.

The product TFA, H-Aib-D-Trp-D-gTrp-CHO was purified by preparative HPLC (Waters, delta pak, C18, 40x100mm, 5µm, 100 A).
Yield =52%.
C₂₆H₃₀N₆O₃, 474 g.mol⁻¹.
¹H NMR (400 MHZ, DMSO-d⁶) + ¹H/¹H correlation : δ 1.21 (s, 3H, CH₃ (Aib)); 1.43 (s, 3H, CH₃ (Aib)); 2.97 (m, 2H, (CH₂)_{β}); 3.1 (m, 2H, (CH₂)_{β'}); 4.62 (m, 1H, (CH)α_{A&B}); 5.32 (q, 0.4H, (CH)α'_{B}); 5.71 (q, 0.6H, (CH)α'_{A}); 7.3 (m, 4H, H₅ and H₆ (2 indoles)); 7.06-7.2 (4d, 2H, H_{2A} et H_{2B} (2 indoles)); 7.3 (m, 2H, H₄ or H₇ (2 indoles)); 7.6-7.8 (4d, 2H, H_{4A} and H_{4B} or H_{7A} et H_{7B}); 7.97 (s, 3H, NH₂ (Aib) and CHO (Formyl)); 8.2 (d, 0.4H, NH_{1B} (diamino)); 8.3 (m, 1H, NH_{A&B}); 8.5 (d, 0.6H, NH_{1A} (diamino)); 8.69 (d, 0.6H, NH_{2A} (diamino)); 8.96 (d, 0.4H, NH_{2B} (diamino)); 10.8 (s, 0.6H, N¹H_{1A} (indole)); 10.82 (s, 0.4H, N¹H_{1B} (indole)); 10.86 (s, 0.6H, N¹H_{2A} (indole)); 10.91 (s, 0.4H, N¹H_{2B} (indole)).
Mass Spectrometry (Electrospray), m/z 475 [M+H]⁺, 949 [2M+H]⁺.

### Analogous synthesis were performed for the following compounds:

### Example 2 H-Aib-D-Mtp-D-gMrp-CHO

C₂₈H₃₄N₆O₃, 502 g.mol⁻¹.
¹H NMR (400 MHZ, DMSO-d⁶) +¹H/¹H correlation : δ 1.19 (s, 2H, (CH₃)_{1A} (Aib)); 1.23 (s,1H, (CH₃)_{1B} (Aib)); 1.41 (s, 2H, (CH₃)_{2A} (Aib)); 1.44 (s, 2H, (CH₃)_{2B} (Aib)); 2.33-2.35 (4s, 6H, 2 CH₃ (indoles)); 2.93 (m, 2H, (CH₂)_{β}); 3.02 (m, 2H, (CH₂)_{β}') ; 4.65 (m, 0.6H, (CH)α_{A}); 4.71 (m, 0.4H, (CH)α_{B}); 5.2 (m, 0.4H, (CH)α'_{B}); 5.6 (m, 0.6H, (CH)α'_{A}); 6.95 (m, 4H, H₅ and H₆ (2 indoles)); 7.19 (m, 2H, H₄ or H₇ (2 indoles)); 7.6 (m, 2H, H₄ or H₇ (2 indoles)); 7.9 (s, 1H, CHO (Formyl)); 7.95 (s, 2H, NH₂ (Aib)); 8.05 (d, 0.4H, NH_{1B} (diamino)); 8.3 (m,1H, NH_{A&B}); 8.35 (m, 0.6H, NH_{1A} (diamino)); 8.4 (d, 0.6H,NH_{2A} (diamino)); 8.75 (d, 0.4H, NH_{2B} (diamino)); 10.69 (s, 0.6H, N¹H_{1A} (indole));10.71 (s, 0.4H, N¹H_{1B} (indole)); 10.80 (s, 0.6H, N¹H_{2A} (indole)); 10.92 (s, 0.4H, N¹H_{2B} (indole)).
Mass Spectrometry (Electrospray), m/z 503.1 [M+H]⁺.

### Example 3 N-Me-Aib-D-Trp-D-gTrp-CHO

Boc-N-Me-Aib-OH (327mg; 1.5mmol; 2.6eq.) was dissolved in methylene chloride (10ml) and cooled to 0°C. Then, dicyclohexylcarbodiimide (156mg; 0.75mmol; 1.3eq.) was added. The mixture, after filtration ofDCU, was added to a solution containing TFA, H-D-Trp-D-gTrp-CHO (0.58mmol; 1eq.) and triethylamine (267µl; 3.3eq.) in methylene chloride (5ml). The reaction mixture was slowly warmed to room temperature and stopped after 24 hr. The mixture was diluted with ethyl acetate (25ml) and washed with saturated aqueous sodium hydrogen carbonate (50ml), aqueous potassium hydrogen sulfate (50ml, 1M), and saturated aqueous sodium chloride (50ml). The organic layer was dried over sodium sulfate, filtered and the solvent removed *in vacuo.* The residue was purified by flash chromatography on silica gel eluting with ethyl acetate/methanol {9/1} to afford 180mg (53%), of Boc-N-Me-Aib-D-Trp-D-gTrp-CHO as a white foam.

Boc-N-Me-Aib-D-Trp-D-gTrp-CHO (180mg; 0.3mmol) was dissolved in a mixture of trifluoroacetic acid (8ml), anisole (1ml) and thioanisole (1ml) for 30 minutes at 0°C. The solvents were removed *in vacuo,* the residue was stirred with ether and the precipitated TFA, N-Me-Aib-D-Trp-D-gTrp-CHO was filtered.

The product TFA, N-Me-Aib-D-Trp-D-gTrp-CHO (39mg; 15%) was purified by preparative HPLC (Waters, delta pak, C18, 40x100mm, 5µm, 100 A).
C₂₇H₃₂N₆O₃, 488 g.mol⁻¹.
¹H RMN (200 MHZ, DMSO-d⁶): δ 1.19 (s, 3H, CH₃ (Aib)); 1.42 (s, 3H, CH₃ (Aib)); 2.26 (s, 3H, NCH₃); 3.12 (m, 4H, 2 (CH₂)_{β}); 4.66 (m, 1H, (CH)_{α}); 5.32 et 5.7 (m, 1H, (CH)_{α'}); 6.9-7.8 (m, 10H, 2 indoles); 8 (m, 1H, CHO (formyl)); 8.2-9 (m, 4H, 3 NH (amides) et NH (amine)); 10.87 (m, 2H, 2 N'H (indoles)).
Mass Spectrometry (Electrospray), m/z 489.29 [M+H]⁺.

### Example 4 H-Aib-D-Trp-D-gTrp-C(O)CH₃

Boc-D-(Nⁱ'Boc)Trp-D-g(NⁱoBoc)Trp-H (0.72mmol; 1eq.) was dissolved in DMF (20ml). Then, N,N-diisopropylethylamine (259ml; 2.leq.) and acetic anhydride (749ml; 1.1eq.) were added. After 1 hr, the mixture was diluted with ethyl acetate (100ml) and washed with saturated aqueous sodium hydrogen carbonate (100ml), aqueous potassium hydrogen sulfate (100ml, 1M), and saturated aqueous sodium chloride (50ml). The organic layer was dried over sodium sulfate, filtered and the solvent removed *in vacuo.* The residue was purified by flash chromatography on silica gel eluting with ethyl acetate/hexane to afford 370mg (73%) of BocD-(NⁱBoc)Trp-D-g(NⁱBoc)Trp-C(O)CH₃ as a white solid.

Boc-D-(N^{l}Boc)Trp-D-g(NⁱBoc)Trp-C(O)CH₃ (350mg, 0.5mmol; 1eq.) was dissolved in a mixture of trifluoroacetic-Acid (8ml), anisole (1ml) and thioanisole (1ml) for 30 minutes at 0°C. The solvents were removed *in vacuo,* the residue was stirred with ether and the precipitated TFA, H-D-Trp-D-gTrp-C(O)CH₃ was filtered.

In 10ml of DMF, TFA, H-D-Trp-D-gTrp-C(O)CH₃ (0.5mmol; 1eq.), Boc-Aib-OH (121mg; 0.59mmol; 1.2eq.), NMM (230µl; 4.2eq.) and BOP (265mg; 1.2eq.) were successively added. After 1 hr, the mixture was diluted with ethyl acetate (25ml) and washed with saturated aqueous sodium hydrogen carbonate (50ml), aqueous potassium hydrogen sulfate (50ml, 1M), and saturated aqueous sodium chloride (50ml). The organic layer was dried over sodium sulfate, filtered and the solvent removed *in vacuo.* The residue was purified by flash chromatography on silica gel eluting with ethyl acetate to afford 249mg (85%) of Boc-Aib-D-Trp-D-gTrp-C(O)CH₃ as a white foam.

Boc-Aib-D-Trp D-gTrp-C(O)CH₃ (249mg; 0.42mmol) was dissolved in a mixture of trifluoroacetic acid (8ml), anisole (1ml) and thioanisole (1ml) for 30 minutes at 0°C. The solvents were removed *in vacuo,* the residue was stirred with ether and the precipitated TFA, H-Aib-D-Trp-D-gTrp-C(O)CH₃ was filtered.

The product TFA, H-Aib-D-Trp D-gTrp-C(O)CH₃ (80mg; 23%) was purified by preparative HPLC (Waters, delta pak, C18, 40x100mm, 5mm, 100 A).
C₂₇H₃₂N₆O₃, 488 g.mol⁻¹.
¹H NMR (200 MHZ, DMSO-d⁶) : δ 1.22 (s, 3H, CH₃ (Aib)); 1.44 (s, 3H, CH₃ (Aib)); 1.8 (s, 3H, C(O)CH₃); 3.06,(m, 4H, 2 (CH₂)_{β}); 4.6 (m. 1H, (CH)_{α}); 5.6 (m, 1H, (CH)_{α}); 6.9-7.8 (m, 10H, 2 indoles); 7.99 (s, 2H, NH₂ (Aib)); 8.2-8.6 (m, 3H, 3 NH (amides)); 10.83 (s,2H, 2 N¹H(indoles)).
Mass Spectrometry (Electrospray), m/z 489.32 [M+H]⁺.

### Example 5 N-Me-Aib-D-Trp-D-gTrp-C(O)CH₃

Boc-N-Me-Aib-OH (1.09g; 5.04mmol; 4eq.) was dissolved in methylene chloride (10ml) and cooled to 0°C. Then, dicyclohexylcarbodiimide (520mg; 2.52mmol; 2eq.) was added. The mixture, after filtration of DCU, was added to a solution containing TFA, H-D-Trp-D-gTrp-C(O)CH₃ (940mg, 1.26mmol; 1eq.) and triethylamine (580ml; 3.3eq.) in methylene chloride (5ml). The reaction mixture was slowly warmed to room temperature and stopped after 24 h. The mixture was diluted with ethyl acetate (SOmI) and washed with saturated aqueous sodium hydrogen carbonate (100ml), aqueous potassium hydrogen sulfate (100ml, 1M), and saturated aqueous sodium chloride (100ml). The organic layer was dried over sodium sulfate, filtered and the solvent removed *in vacuo*. The residue was purified by flash chromatography on silica gel eluting with ethyl acetate/methanol {9/1} to afford 530mg(70%) of Boc-N-Me-Aib-D-Trp-D-gTrp-C(O)CH₃ as a white foam.

Boc-N-Me-Aib-D-Trp-D-gTrp-C(O)CH₃ (530mg; 0.88mmol) was dissolved in a mixture of trifluoroacetic acid (8ml), anisole (1ml) and thioanisole (1ml) for 30 minutes at 0°C. The solvents were removed *in vacuo,* the residue was stirred with ether and the precipitated TFA, N-Me-Aib-D-Trp-D-gTrp-C(O)CH₃ was filtered.

The product TFA, N-Me-Aib-D-Trp-D-gTrp-C(O)CH₃ (220mg; 30%) was purified by preparative HPLC (Waters, delta pak, C18, 40x100mm, 5mm, 100 A).
C₂₆H₃₄N₆O₃,502 g.mol⁻¹.
¹H NMR (200 MHZ, DMSO-d⁶) : δ 1.17 (s,3H, CH₃ (Aib)); 1.4 (s, 3H, CH₃ (Aib)); 1.78 (s, 3H, C(O)CH₃); 2.23 (s, 3H, NCH₃); 3.15 (m, 4H, 2 (CH₂)_{β}); 4.7 (m, 1H, (CH)_{α}); 5.55 (m, 1H, (CH)_{α}); 6.9-7.9 (m, 10H, 2 indoles); 8.2-8.8 (s, 4H, NH (amine) et 3 NH (amides)); 10.8 (s, 2H, 2N¹H (indoles)).
Mass Spectrometry (Electrospray), m/z 503.19 [M+H]⁺.

### Examples 10-62

The following compounds were prepared in similar manners:

### Example 10 H-Aib-D-Mrp-gMrp-CHO

### Example 11 H-Aib=Trp-gTrp-CHO

### Example 12 H-Aib-Trp-D-gTrp-CHO

### Example 13 H-Aib-D-Trp-gTrp-CHO

### Example 19 Aib-D-Trp-gTrp-CO-CH₂-CH₃

### Example 20 Aib-D-Trp-gTrp-CO-CH₂-CH(CH₃)-CH₃

### Example 21 Aib-D-Trp-gTrp-CO-CH₂-phenyl

### Example 23 Aib-D-Trp-gTrp-CO-CH₂-pyrrol-3-yl

### Example 24 Aib-D-Trp-gTrp-CO-CH₂-CH₂-cyclohexyl

### Example 25 N-Me-Aib-D-Trp-gTrp-CO-CH₂-CH₃

### Example 26 N-Me-Aib-D-Trp-gTrp-CO-CH₂-CH(CH₃)-CH₃

### Example 27 N-Me-Aib-D-Trp-gTrp-CO-CH₂-phenyl

### Example 28 N-Me-Aib-D-Trp-gTrp-CO-CH₂-pyrrol-3-yl

### Example 29 N-Me-Aib-D-Trp-gTrp-CO-CH₂-CH₂-cyclohexyl

### Example 30 Aib-D-Trp-gTrp-CHO

### Example 40 N-Me-Aib-D-Trp-N-Me-D-gTrp-C(O)CH₃

### Example 41 H-Aib-D-Trp-N-Me-D-gTrp-C(O)CH₃

### Example 52 N(Me)₂-Aib-(D)-Trp-(D)-gTrp-formyl

C₂₈H₃₆N₆O₃,502 g.mol⁻¹.
¹H RMN (400 MHz, DMSO-d⁶): δ 1.2 (s, 3H, CH₃ (Aib)); 1.39 (s, 3H, CH₃ (Aib)); 2.29 (m, 3H,NCH₃); 2.99-3.33 (m, 4H, 2 (CH₂)_{β}); 4.68 (m,1H, CH)_{α}) ; 5.3 and 5.69 (m, 1H, CH)_{α'}) ; 6.97-7.72 (m, 10H, 2 indoles) ;7.97 (2s, 1H, formyl); 82-9.47 (m, 3H, 3 NH (amides)); 10.85 (m,2H,2 NH(indoles)).
Mass Spectrometry (Electrospray), m/z 503.45 [M+H]⁺.
Analytic HPLC (Symmetry shield 3.5µ C18 100A, 1ml/min, 214nm, eluent: H₂O / ACN 0.1% TFA, gradient 0 to 100% ACN in 15min), tr = 6.63 min, 99%. Freezedried Compound.

### Example 53 N(Me)₂-Aib-D-Trp-D-gTrp-acetyl

C₂₉H₃₆N₆O₃, 516 g.mol⁻¹.
¹H RMN (200 MHz, DMSO-d⁶): δ 1.22 (s, 3H, CH₃(Aib)); 1.4 (s, 3H, CH₃ (Aib)); 1.8 (s, 3H, acetyl) ; 2.28 (d, 3H, NCH₃) ; 2.96-3.22 (m, 4H, 2 (CH₂)_{β}); 4.7 (m, 1H, (CH)_{α}) ; 5.60 (m, 1H, (CH)_{α}); 6.98-7.75 (m, 10H, 2 indoles) ; 8.2-9.47 (m, 3H, 3 NH (amides)); 10.84 (m, 2H, 2 NH (indoles)).
Mass Spectrometry (Electrospray), m/z 517.34 [M+H]⁺.
Analytic HPLC (Symmetry shield 3.5µ C18 100A, 1ml/min, 214nm, eluent: H₂O / ACN 0.1% TFA, gradient 0 to 100% ACN in 15min), tr= 7.07mm, 99%. Freezedried Compound.

### Example 60 H-Aib-(R)-Mrp-(D)-gTrp-formyl

### Example 61 H-Aib-(D)-Trp-(R)-gMrp-formyl

### Example 62 N-Me-Aib-(D)-Trp-(R)-gMrp-acetyl

### Example 63 EVALUATION OF THE GROWTH HORMONE RELEASING ACTIVITY OF NEW GROWTH HORMONE SECRETAGOGUES IN THE INFANT RAT

### Animals

Male 10-day-old Sprague Dawley rats, about 25 g body weight were used.

Pups were received on the fifth day after birth and were housed under controlled conditions (22 ± 2°C, 65 % humidity and artificial light from 06.00 to 20.00 h). A standard dry diet and water were available ad libitum to the dams.

### Experimental procedure

One hour before the experiments, pups were separated from their respective dams and were divided randomly into groups of eight each.

Pups were acutely challenged subcutaneously with 100 µl of solvent (DMSO, final dilution 1:300 in physiological saline), hexarelin
(Tyr-Ala-His-D-Mrp-Ala-Trp-D-Phe-Lys-NH₂, used as a reference drug), or new compounds (300 µg/kg) and killed by decapitation 15 min later.

Trunk blood was collected and centrifuged immediately: Plasma samples were stored at -20°C until assayed for the determination of plasma GH concentrations.

Growth hormone concentrations in plasma were measured by RIA using materials provided by the National Institute of Diabetes, Digestive and Kidney Diseases (NIDDK) of the National Institute of Health U.S.A.

Values were expressed in terms of the NIDDK-rat-GH-RP-2 standard (potency 21U/mg) as ng/ml of plasma.

The minimum detectable value of rat GH was about 1.0 ng/ml, and intraassay variability was about 6 %.

The obtained results of several test series, wherein the in vivo activity in the rat was determined, are listed in tables 1 to 10.

**Table 1**

| Example | Structure | GH ng/ml |
|---|---|---|
| 1 | H-Aib-D-Trp-D-gTrp-CHO | 158.8 ± 39.4 |
| 13 | H-Aib-D-Trp-gTrp-CHO | 58 ± 6.3 |
| SOLVENT | | 15.0 ± 8.0 |
| HEXARELIN | Tyr-Ala-His-D-Mrp-Ala-Trp-D-Phe-Lys-NH₂ | 202 ± 32.7 |

**Table 2**

| Example | Structure | GH ng/ml |
|---|---|---|
| 3 | N-Me-Aib-D-Trp-D-gTrp-CHO | 86.6 ± 12.6 |
| 4 | H-Aib-D-Trp-D-gTrp-C(O)CH₃ | 104.7 ± 13.5 |
| 5 | N-Me-Aib-D-Trp-D-gTrp-C(O)CH₃ | 175.5 ± 37.2 |
| SOLVENT | | 20.7 ± 0.9 |
| HEXARELIN | Tyr-Ala-His-D-Mrp-Ala-Trp-D-Phe-Lys-NH₂ | 134.5 ± 27.2 |

**Table 3**

| Example | Structure | GH ng/ml |
|---|---|---|
| 19 | Aib-D-Trp-gTrp-CO-CH₂-CH₃ | 79.8 ± 22.4 |
| 20 | Aib-D-Trp-gTrp-CO-Piperidin-4-yl | 153.6 ± 30.6 |
| SOLVENT | | 22.3 ± 5 |
| HEXARELIN | Tyr-Ala-His-D-Mrp-Ala-Trp-D-Phe-Lys-NH₂ | 114.7 ± 8.4 |

**Table 4**

| Example | Structure | GH ng/ml |
|---|---|---|
| 40 | N-Me-Aib-D-Trp-N-Me-D-gTrp-C(O)CH₃ | 188.2 ± 28.5 |
| 41 | H-Aib-D-Trp-N-Me-D-gTrp-C(O)CH₃ | 75.4 ± 15.0 |
| SOLVENT | | 10.55 ± 2.65 |
| HEXARELIN | Tyr-Ala-His-D-Mrp-Ala-Trp-D-Phe-Lys-NH₂ | 114.5 ± 12.9 |

**Table 7**

| Example | Structure | GH ng/ml |
|---|---|---|
| 52 | N(Me)₂-Aib-(D)-Trp-(D)-gTrp-formyl | 121.43 ± 29 |
| 53 | N(Me)₂-Aib-(D)-Trp-(D)-gTrp-acetyl | 26.80 ± 5.64 |
| SOLVENT | | 7.89 ± 1.77 |
| HEXARELIN | | 172.5 ± 38.53 |

**Table 8**

| Example | Structure | GH ng/ml |
|---|---|---|
| 60 | H-Aib-(R)Me-Trp-(D)-gTrp-formyl | 21.02 ± 3.43 |
| 61 | H-Aib-(D)-Trp-(R)-Me-gTrp-formyl | 152.28 ± 43.76 |
| 62 | H-Me-Aib-(D)-Tip-(R)-Me-gTrp-acetyl | 171.78 ± 10.32 |
| SOLVENT | | 7.89 ± 1.77 |
| HEXARELIN | | 172.5 ± 38.53 |

Furthermore the time dependence of the oral activity in the dog (1mg/kg; per os) was estimated for example 1. (H-Aib-D-Trp-D-gTrp-CHO). Well-trained beagles of either sex, > 10 year, 10-15 kg by weight, were used. Animals were fed normal dry food with water ad libitum and were on a 12b-light/12h-dark regimen with on at 7.00. The compound was administered orally to the dogs which had fasted since 16.00 of the preceding day. Blood samples were taken 20 min before administration, at administration and 15, 30, 60, 90, 120 and 180 min after administration. The results are given in table 11.

**Table 11**

| Example | NAME OF THE DOG | | | | | | | |
|---|---|---|---|---|---|---|---|---|
| 1 | DAKOTA | JORMA | RAZ DEGAN | FORREST LEE | MARKUS | TAYLOR | MEAN VALUE | SEM |
| t (min) | Concentration GH (ng/ml) | | | | | | | |
| | | | | | | | | |
| -20 | 0.48 | 3.58 | 2.14 | 1.43 | 2.45 | 2.32 | 2.07 | 0.38 |
| 0 | 0.35 | 2.75 | 1.64 | 2.01 | 2.55 | 1.41 | 1.79 | 1.03 |
| 15 | 2.11 | 8.91 | 3.58 | 6.38 | 6.11 | 4.8 | 5.32 | 1.02 |
| 30 | 0.54 | 6.85 | 6.37 | 8.48 | 6.9 | 3.89 | 5.5 | 1.07 |
| 60 | 0.17 | 2.65 | 3.02 | 4.41 | 6.51 | 4.34 | 3.52 | 0.84 |
| 90 | 0.4 | 2.47 | 2.61 | 6.42 | 5.18 | 4.43 | 3.59 | 0.66 |
| 120 | 3.58 | 2.48 | 1.94 | 3.71 | 4.54 | 4.28 | 3.42 | 0.38 |
| 180 | 3.46 | 2.82 | 1.49 | 3.18 | 4.12 | 3.18 | 3.04 | 0.36 |
| | | | | | | | | |
| AUC | 328.53 | 658.38 | 510.64 | 888.91 | 944.26 | 721.34 | 675.35 | 94.47 |

| | | | | | | | | |
|---|---|---|---|---|---|---|---|---|
| SEM = Standard deviation AUC = Area under the curve | | | | | | | | |

## Claims

1. Compounds of the formula I 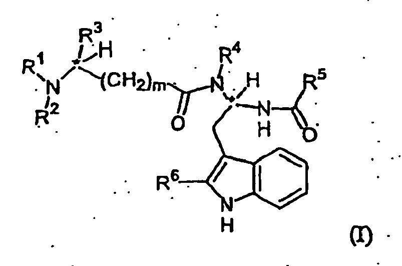 wherein * means a carbon atom which, when a chiral carbon atom, has a R or S configuration, R¹ is an hydrogen atom and R³ is a group of formule II 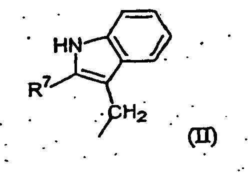 R² is a group according to formula IV ; 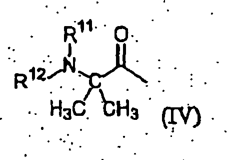 R⁴ is a hydrogen atom or a linear or branched C₁-C₄-alkyl group, R⁵ is a hydrogen atom, a linear or branched C₁-C₄ alkyl group, a (CH₂)ₙ-aryl group, wherein aryl is phenyl or napthyl, a (CH₂)ₙ-heterocyclic group, wherein the heterocyclic group is isonipecotyl, 4-piperidinyl or 3-pyrrolyl, a (CH₂)ₙ-cycloalkyl group, wherein the cycloalkyl group is cyclohexyl, or an amino group, R₆ and R₇ are independently from each other a hydrogen atom or a linear or branched C₁-C₄-alkyl group, R₁₁ and R₁₂ are independently from each pother a hydrogen atom or a linear or branched C₁-C₄-alkyl group, m is 0 and n is 1 or 2.

2. Compounds according to claim 1, wherein the linear or branched C₁-C₄ alkyl group is methyl.

3. A compound which is

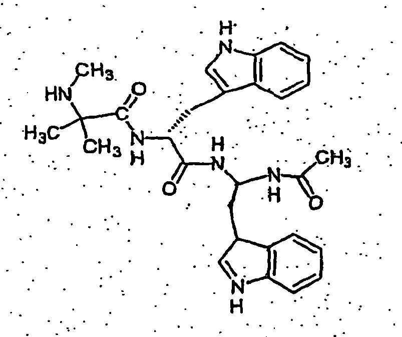

4. A compound which is

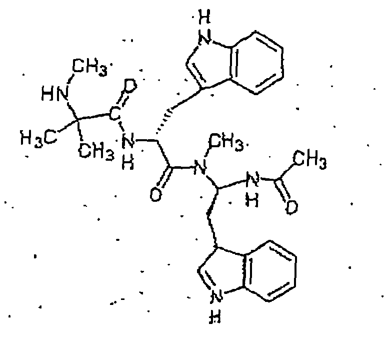

5. A pharmaceutical composition, comprising a compound of claim 1.

6. The composition of claim 5, in combination with a pharmaceutically acceptable carrier.

7. The composition of claim 5, in combination with an additional growth hormone secretagogue.

8. The use of a compound according to claim 1 for the manufacture of a medicament for elevating the plasma level of growth hormone in a mammal.

9. The use of a compound according to claim 1 for the manufacture of a medicament for the treatment of growth hormone secretion deficiency.

10. The use of a compound according to claim 1 for the manufacture of a medicament for the treatment of growth retardation in a child.

11. The use of a compound according to claim 1 for the manufacture of a medicament for the treatment of metabolic disorders associated with growth hormone secretion deficiency in a subject.

12. The use as claimed in Claim 11 wherein the subject is an aged subject.

13. The use of a compound according to claim 1 for the manufacture of a medicament for promoting wound healing, recovery from surgery or recovery from debilitating illnesses.

## Patentansprüche

1. Verbindung der Formel I, 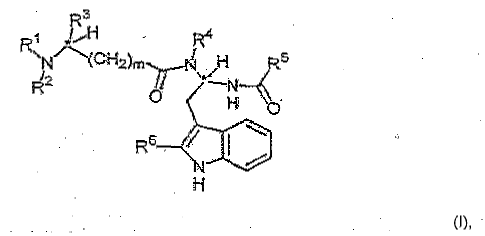 wobei * ein Kohlenstoffatom bedeutet, das, wenn es ein chirales Kohlenstoffatom ist, eine R. oder,S-Konfiguration ist, R¹ ein Wasserstoffatom ist und R³ eine Gruppe der Formel II ist. 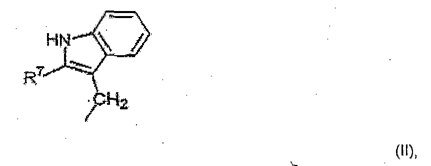 R² eine Gruppe gemäß der Formel IV ist: 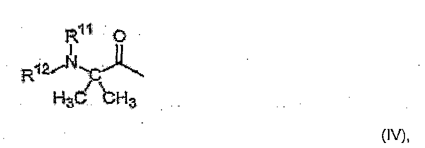 R⁴ ein Wasserstoffatom oder eine lineare oder verzweigte C₁-C₄-Alkylgruppe ist, R⁵ ein Wasserstoffatom, eine lineare oder verzweigte C₁-C₄-Alkylgruppe, eine (CH₂)ₙ-Arylgruppe, wobei Aryl, Phenyl oder Napthyl ist, eine (CH₂)ₙ-heterozyklische Gruppe, wobei die heterozyklische Gruppe isonipecotyl, 4-Piperidinyl oder 3-Pyrrolyl ist, eine (CH₂)ₙ-Zykloalkylgruppe, wobei die Zykloalkylgruppe Zyklohexyl ist, oder eine Aminogruppe ist, R⁶ und R⁷ unabhängig voneinander ein Wasserstoffatom oder eine lineare oder verzweigte C₁-C₄-Alkylgruppe sind, R₁₁, und R₁₂ unabhängig voneinander ein Wasserstoffatom oder eine lineare oder verzweigte C₁-C₄-Alkylgruppe sind, m 0 ist und n 1 oder 2 ist.

2. Verbindung nach Anspruch 1, wobei die lineare oder verzweigte C₁-C₄-Alkylgruppe Methyl ist.

3. Verbindung, bei der es sich handelt um:

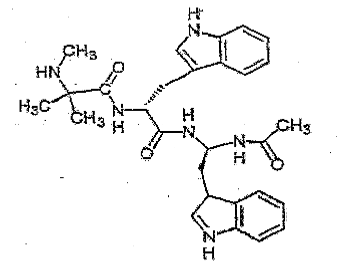

4. Verbindung, bei der es sich handelt um:

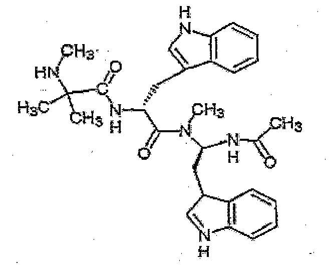

5. Pharmazeutische Zusammensetzung, die eine Verbindung nach Anspruch 1 aufweist.

6. Zusammensetzung nach Anspruch 5 in Kombination mit einem pharmazeutisch akzeptablen Träger.

7. Zusammensetzung nach Anspruch 5 in Kombination mit einem Wachstumshormonsekretion fördernden Mittel.

8. Verwendung einer Verbindung nach Anspruch 1 bei der Herstellung eines Medikaments zur Erhöhung des Plasmaniveaus von Wachstumshormonen bei einem Säugetier.

9. Verwendung einer Verbindung nach Anspruch 1 für die Herstellung eines Medikaments für die Behandlung von Wachstumshormonsekretionsdefizienzen.

10. Verwendung einer Verbindung nach Anspruch 1 für die Herstellung eines Medikaments für die Behandlung einer Wachstumsverzögerung bei einem Kind.

11. Verwendung einer Verbindung nach Anspruch 1 für die Herstellung eines Medikaments zur Behandlung von Stoffwechselstörungen, die mit einer Wachstumshormonsekretionsdefizienz bei einem Subjekt verbunden sind.

12. Verwendung nach Anspruch 11, wobei das Subjekt ein gealtertes Subjekt ist.

13. Verwendung einer Verbindung nach Anspruch 1 für die Herstellung eines Medikaments zur Förderung der Wundheilung, Erholung von chirurgischen Eingriffen oder Erholung von schwächenden Erkrankungen.

## Revendications

1. Composés de formule I 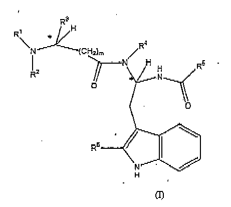 dans laquelle * signifie un atome de carbone qui, lorsqu'il est un atome de carbone chiral, a une configuration R ou S, R¹ est un atome d'hydrogène et R³ est un groupe de formule II 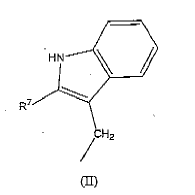 R² est un groupe selon la formule IV 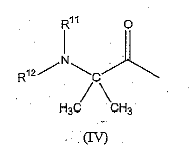 R⁴ est un atome d'hydrogène ou un groupe alkyle en C₁-C₄, linéaire ou ramifié, R⁵ est un atome d'hydrogène, un groupe alkyle en C₁-C₄, linéaire ou ramifié, un groupe (CH₂)ₙ-aryle, dans lequel aryle est un phényle ou un naphtyle, un groupe (CH₂)ₙ-hétérocyclique, dans lequel le groupe hétérocyclique est un isonipécotyle, 4-pipéridinyle ou 3-pyrrolyle, un groupe (CH₂)ₙ-cycloalkyle, dans lequel le groupe cycloalkyle est un cyclohexyle, ou un groupe amino, R₆ et R₇ sont, indépendamment l'un de l'autre, un atome d'hydrogène ou un groupe alkyle en C₁-C₄, linéaire ou ramifié, R₁₁ et R₁₂ sont indépendamment l'un de l'autre un atome d'hydrogène ou un groupe alkyle en C₁-C₄, linéaire ou ramifié, m est 0 et n est 1 ou 2.

2. Composés selon la revendication 1, dans lequel le groupe alkyle en C₁-C₄, linéaire ou ramifié, est un méthyle.

3. Composé qui est

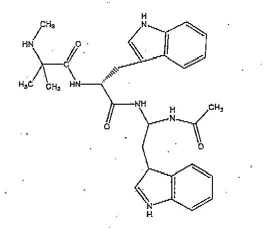

4. Composé qui est

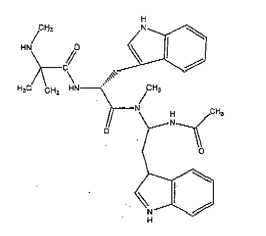

5. Composition pharmaceutique, comprenant un composé selon la revendication 1.

6. Composition selon la revendication 5, en combinaison avec un véhicule pharmaceutiquement acceptable.

7. Composition selon la revendication 5, en combinaison avec un sécrétagogue supplémentaire de l'hormone de croissance.

8. Utilisation d'un composé selon la revendication 1, pour la fabrication d'un médicament pour élever le taux plasmatique d'hormone de croissance chez un mammifère.

9. Utilisation d'un composé selon la revendication 1, pour la fabrication d'un médicament pour le traitement d'un déficit de sécrétion d'hormone de croissance.

10. Utilisation d'un composé selon la revendication 1, pour la fabrication d'un médicament pour le traitement d'un retard de croissance chez un enfant.

11. Utilisation d'un composé selon la revendication 1, pour la fabrication d'un médicament pour le traitement de troubles métaboliques associés à un déficit de sécrétion d'hormone de croissance chez un sujet.

12. Utilisation selon la revendication 11, dans laquelle le sujet est un sujet âgé.

13. Utilisation d'un composé selon la revendication 1, pour la fabrication d'un médicament pour activer la cicatrisation d'une blessure, le rétablissement d'une opération chirurgicale ou le rétablissement de maladies débilitantes.
